# EUROPÄISCHE PATENTANMELDUNG

(11) **EP 1 123 873 A2**
(43) Veröffentlichungstag der Anmeldung: **16.08.2001**
(21) Anmeldenummer: 01103047.5
(22) Anmeldetag: 09.02.2001
(51) Int. Cl.: B65D 50/06

(54) **Kindersichere Austragvorrichtung für Medien**

(30) Priorität: 12.02.2000 DE 10006370
(71) Anmelder: Ing. Erich Pfeiffer GmbH, 78315 Radolfzell (DE)
(72) Erfinder: Fuchs, Karl-Heinz, 78315 Radolfzell (DE)
(74) Vertreter: Patentanwälte , Ruff, Wilhelm, Beier, Dauster & Partner

(57) **Zusammenfassung**

Aufgabe der Erfindung ist es, eine Verschlußkappe für Austragvorrichtungen für Medien zu schaffen, die nicht unbeabsichtigt und auch nicht von Kindern öffenbar ist.

Eine erfindungsgemäße Austragvorrichtung für Medien weist ein Gehäuse (11) mit einer Austragöffnung (12) zum Austragen des Mediums auf. Bei dem Medium handelt es sich vorzugsweise um ein Fluid, das insbesondere einen pharmazeutischen Wirkstoff enthalten kann. Auf dem Gehäuse (11) durch Verrasten befestigbar ist eine Verschlußkappe (20), die die Austragöffnung (12) überdeckt. Dabei ist die Verrastung zwischen Gehäuse (11) und Verschlußkappe (20) so ausgebildet, daß ein Abnehmen der Verschlußkappe (20) nur durch eine Betätigung der Verschlußkappe (20) möglich ist, die wenigstens zwei Teilbetätigungen umfaßt. Dabei erfolgen die Teilbetätigungen unmittelbar aufeinander. Die Teilbetätigungen unterscheiden sich im wesentlichen dadurch, daß jede Teilbetätigung eine von der vorherigen Teilbetätigung unabhängige Betätigungsrichtung erfordert.

## Beschreibung

Die Erfindung betrifft Austragvorrichtungen für Medien, insbesondere für das Versprühen eines vorzugsweise wenigstens einen pharmazeutischen Werkstoff enthaltenden Fluids.

Austragvorrichtungen für Medien gemäß dem Oberbegriff des Anspruches 1 sind vielfältig bekannt. Eine derartige Austragvorrichtung für Medien geht beispielsweise aus der DE 197 53 147 A1 bekannt.

Eine gattungsgemäße Austragvorrichtung für Medien ist insbesondere für das Versprühen eines vorzugsweise einen pharmazeutischen Wirkstoff enthaltenden Fluids, beispielsweise Migränemittel oder andere Medikamente, die durch Versprühen in der Nase oder im Mund des Patienten appliziert werden können, geeignet. Derartige Austragvorrichtungen weisen ein Gehäuse auf. Dieses Gehäuse weist eine an den Applikationspunkt anzusetzende Austrittsöffnung auf. Mit einer Verschlußkappe wird bei Nichtgebrauch die Austragvorrichtung insbesondere im Bereich der Austragöffnung verschlossen. Dies geschieht, um zu verhindern, daß in unnötiger Weise Keime zusammen mit der Umgebungsluft zur Austragöffnung gelangen können. Es soll somit durch das Verschließen der Austragöffnung mit einer Verschlußkappe eine unnötig hohe oder sogar für Produkt oder Patient gefährliche Verschmutzung der Austragöffnung, insbesondere von der Umgebungsluft her, verhindert werden. Damit die Verschlußkappe auf dem Gehäuse gehalten wird, ist eine Verrastung zwischen Verschlußkappe und Gehäuse vorzusehen.

Eine Verschlußkappe, die lediglich durch Verrasten verschlossen wird, ist leicht abnehmbar. Sie kann, beispielsweise wenn sie transportiert wird, auch durch den Einfluß anderer Gegenstände im gleichen Transportbehältnis, zum Beispiel einer Handtasche, von dem Gehäuse getrennt werden. Dies ist insbesondere bei sehr empfindlichen Medikamenten nicht erwünscht. Gleichzeitig können in der Ausgabevorrichtung auch Medikamente enthalten sein, die aufgrund ihrer Wirkstoffzusammensetzung oder aus anderen Gründen nur bewußt abgegeben werden sollen. Insbesondere ist zu verhindern, daß dann, wenn die Austragvorrichtung Kindern in die Hände gelangt ist, diese die Verschlußkappe öffnen und somit sich den Wirkstoff applizieren können.

Aufgabe der Erfindung ist es daher, eine Verschlußkappe für Austragvorrichtungen für Medien zu schaffen, die nicht unbeabsichtigt und auch nicht von Kindern öffenbar ist.

Diese Aufgabe wird bei Zugrundelegen der gattungsgemäßen Austragvorrichtung für Medien erfindungsgemäß durch eine Austragvorrichtung mit den Merkmalen des kennzeichnenden Teils des Anspruchs 1 gelöst.

Eine erfindungsgemäße Austragvorrichtung für Medien weist ein Gehäuse mit einer Austragöffnung zum Austragen des Mediums auf. Bei dem Medium handelt es sich vorzugsweise um ein Fluid, das insbesondere einen pharmazeutischen Wirkstoff enthalten kann. Auf dem Gehäuse durch Verrasten befestigbar ist eine Verschlußkappe, die die Austragöffnung überdeckt. Dabei ist die Verrastung zwischen Gehäuse und Verschlußkappe so ausgebildet, daß ein Abnehmen der Verschlußkappe nur durch eine Betätigung der Verschlußkappe möglich ist, die wenigstens zwei Teilbetätigungen umfaßt. Dabei erfolgen die Teilbetätigungen unmittelbar aufeinander. Die Teilbetätigungen unterscheiden sich im wesentlichen dadurch, daß jede Teilbetätigung eine von der vorherigen Teilbetätigung unabhängige Betätigungsrichtung erfordert.

Vorzugsweise erfolgt dabei die erste Teilbetätigung in einer Betätigungsrichtung, die der Richtung dem Entfernen der Verschlußkappe vom Gehäuse entgegengesetzt ist.

Weiter bevorzugt wird eine Ausgestaltung, bei der die zweite Teilbetätigung eine Rotation der Verschlußkappe um ihre Längsachse ist. Der Drehwinkel der Rotation der zweiten Teilbetätigung ist dabei begrenzt, er bewegt sich vorzugsweise in einem Winkel kleiner 90°, insbesondere zwischen 10 und 45°.

Gemäß vorteilhafter Ausgestaltung ist die Verrastung zwischen Verschlußkappe und Gehäuse als Kulissenführung ausgebildet. Vorzugsweise ist dabei die Kulisse der Kulissenführung in der Verschlußkappe ausgebildet. Weiter bevorzugt wird eine Ausgestaltung der Erfindung, in der der Verlauf der Kulisse dem Kulissenverlauf bei Bajonettverschlüssen entspricht.

Gemäß weiter bevorzugter Ausgestaltung der Erfindung sind zwischen Verschlußkappe und Gehäuse mehrere regelmäßig zueinander beabstandete Kulissenführungen ausgebildet.

Gemäß weiter bevorzugter Ausgestaltung sind zwischen Verschlußkappe und Gehäuse wirkende Kraftspeicher vorgesehen. Die Kraftspeicher wirken dabei im Sinne eines Aufrechterhaltens der Verrastung zwischen Verschlußkappe und Gehäuse. Gemäß bevorzugter Ausgestaltung sind die Kraftspeicher aus an der Verschlußkappe ausgebildeten, elastisch verformbaren Stegen gebildet, die mit einer entsprechend geformten Fläche des Gehäuses zusammenwirken.

Weiter vorteilhaft ist es, wenn an der Verschlußkappe Wirkelemente ausgebildet sind, die der zweiten Teilbetätigung der Verschlußkappe entgegenwirken.

Weiter bevorzugt wird eine zweiteilige Ausgestaltung der Verschlußkappe, wobei ein Innenteil in einem Außenteil befestigt ist und wobei das Außenteil eine geschlossene, eine Öffnung zur Aufnahme des Gehäuses aufweisende Oberfläche besitzt. Vorzugsweise sind Kulissenführungen und/oder Kraftspeicher und/oder Wirkelemente an dem Innenteil ausgebildet. Dabei ist vorzugsweise an der Verschlußkappe, besonders bevorzugt am Außenteil der Verschlußkappe, ein Formabschnitt ausgebildet, der die Austragöffnung umschließt und dichtend am Gehäuse anliegt.

Die Unteransprüche enthalten vorteilhafte Weiterbildungen der Ausgestaltungen gemäß den unabhängigen Patentansprüchen. Im übrigen ist die Erfindung auch anhand der in der Zeichnung dargestellten Ausführungsbeispiele noch näher erläutert; dabei zeigen:
- Fig. 1: die Teilschnitt-Darstellung einer erfindungsgemäßen Austragvorrichtung für Medien;
- Fig. 2a bis Fig. 2e: die im Betätigungselement angeordnete Steuereinheit mit der ansteuerbaren Betätigungssperre in Freigabestellung und in Sperrstellung;
- Fig. 3a bis Fig. 3c: die Darstellung einer erfindungsgemäßen Verschlußkappe mit Kulissenführung;
- Fig. 4a und Fig. 4b: die Ausgestaltung eines gravitationsabhängig schaltenden Sperrmittels in einer die Betätigung ermöglichenden Stellung und in einer die Betätigung verhindernden Stellung; und
- Fig. 5: ein Blockschaltbild einer Steuereinheit und der von ihr betätigbaren Betätigungssperre.

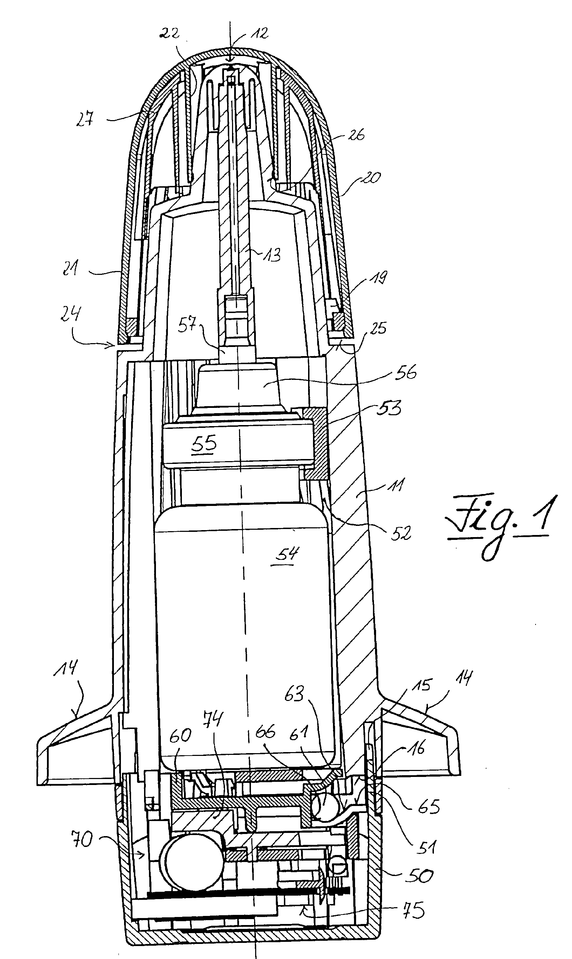

Die Fig. 1 zeigt in teilgeschnittener Darstellung eine Austragvorrichtung für Medien, wie sie insbesondere zum Versprühen eines Fluids, das vorzugsweise einen pharmazeutischen Wirkstoff enthält, verwendet werden kann.

Die Austragvorrichtung weist ein Gehäuse 11 auf. In dem Gehäuse sind, nach außen verschlossen, die funktionalen Elemente angeordnet. Das Gehäuse weist zum Austragen des Mediums, insbesondere eines Fluids, eine Austragöffnung 12 auf. Damit eine Verschmutzung im Bereich der Austragöffnung 12 und somit auch eine eventuelle Verkeimung oder eine Verseuchung mit Bakterien möglichst vermieden wird, wird das Gehäuse mit der verrastend auf ihm aufsetzbaren Verschlußkappe 20 verschlossen. Die Verschlußkappe 20 wird dabei aus einem Außenteil 21 und einem Innenteil 27 gebildet. Dabei verbleibt zwischen dem unteren Rand der Verschlußkappe 25 und dem zugeordneten Gehäuseabschnitt ein schmaler Spalt 24. Ansonsten bildet Gehäuse 11 zusammen mit der aufgesetzten Verschlußkappe 20 eine weitgehend gleichmäßige und geschlossene Oberfläche aus. Das Außenteil 21 der Verschlußkappe weist auch noch den Formabschnitt, der hier die Form eines Hohlzylinders hat und von innen an der geschlossenen Fläche 23 des Außenteils 21 angeordnet ist und in das Innenteil 27 der Verschlußkappe 20 hineinragt. Der Formabschnitt 22 umschließt das Gehäuse 11 in dem Bereich, der die Austragöffnung 12 beinhaltet und liegt im Bereich der Anlagefläche 26 dichtend am Gehäuse an. Dadurch wird sichergestellt, daß keine Luft von außen mit dem im hohlzylindrischen Abschnitt des Formabschnitts 22, der durch die dichtende Anlage 26 am Gehäuse verschlossen wird, ausgetauscht werden kann. Dadurch wird einer Verschmutzung bzw. einer Verkeimung des Gehäuses 11 im Bereich der Austragöffnung 12 verhindert. Das Innenteil 27 der Verschlußkappe 20 weist eine Kulissenführung 28 für den Gleitstein 19, der am Gehäuse 11 ausgebildet ist, auf. Die Kulissenführung ist in dieser teilgeschnittenen Darstellung nicht ersichtlich.

An seinem der Verschlußkappe 20 abgewandten Ende weist das Gehäuse 11 Griffflächen 14 auf, an denen während der Benutzung der Austragvorrichtung das Gehäuse 11 gehalten werden kann. An seinem unteren Ende ist das Gehäuse geöffnet und wird durch das Betätigungselement 50 verschlossen. Zur Führung des Betätigungselementes 50 im Gehäuse 11 ist die Ringnut 15 im Gehäuse ausgebildet, in die bei der Betätigung der entsprechende Tauchsteg 51 des Betätigungselementes eintaucht. Die Strecke eines Betätigungshubes des Betätigungselementes wird dabei im Zusammenwirken zwischen der Tiefe der Ringnut 15 des Gehäuses 11 und der Länge des Tauchsteges 51 am Betätigungselement 50 festgelegt. In der gewählten Ausführung der Erfindung ist der Betätigungshub dadurch begrenzt, daß der Tauchsteg 51 am Boden der Ringnut 15 anstößt. In nicht betätigter Stellung des Betätigungselementes 50 ist daher ein Spalt zwischen Betätigungselement 50 und Gehäuse 11 vorhanden. Dieser Spalt kann mit einem Originalitätsschutz verschlossen werden, der Sollbruchstellen aufweist und daher entfernbar ist.

Lagefest mit dem Gehäuse 11 ist, zur Austragöffnung 12 führend und den Austragkanal beinhaltend, das Distanzstück 13 angeordnet. Die Länge des Distanzstückes 13 bestimmt sich aus dem im Gehäuse 11 verbleibenden Platz in Abhängigkeit mit der Größe des Behälters 54. Je größer der Behälter 54 gewählt wird, desto mehr Betätigungen der Betätigungselemente 50 können durchgeführt werden, bevor der Behälter 54 entleert ist. Das Distanzstück 13 ist dabei lagefest zum Gehäuse 11 angeordnet. Im Gegensatz dazu ist der Behälter 54 lagefest am Betätigungselement 50 angeordnet. Der Behälter 54 ist mittels des Crimp-Ringes 55 durch die Saugpumpe 56 verschlossen. Der Kolbenstößel 57 der Saugpumpe 56 ist dabei so ausgebildet, daß er sich in Anlage mit dem Distanzstück 13 befindet, wobei zum Austragen des auszutragenden Mediums ein durchgehender Kanal durch den Kolbenstößel 57 und das Distanzstück 13 hindurch zur Austragöffnung 12 ausgebildet ist. Der Behälter 54 ist selbst über den am Crimp-Ring 55 angreifenden Halter 53 an dem Tragelement 52 befestigt. Das Tragelement 52 selbst ist wiederum, zumindest mittelbar, an dem Betätigungselement 50 befestigt.

Im Bereich des unteren Abschlusses des Gehäuses 11, der durch die Anschlagfläche 16 gebildet wird, ist im Betätigungselement 50, als mechanisch ausgeführtes Schaltmittel eines gravitationsabhängig schaltenden Sperrmittels, der Führungsring 60 angeordnet. In dem Führungsring 60 sind mehrere, in dem dargestellten Beispiel drei jeweils um 120° zueinander versetzte Kurvenbahnen 62 eingebracht, in denen eine Kugel 61 geführt ist. In der Darstellung der Fig. 1 ist aber lediglich eine Kurvenbahn 62 ersichtlich. Die Kurvenbahn 62 weist an ihrem inneren Ende die Anschlagfläche 66 auf, an der in dieser Ausrichtung der Austragvorrichtung die Kugel 61 anliegt. In dieser Stellung ermöglicht die Kugel die Durchführung eines Betätigungshubes des Betätigungselementes 50. Die Kurvenbahn weist außer ihrem Endanschlag 66 eine Rampe in Form einer schiefenen Ebene 63 auf, die gegenüber der Horizontalen den Neigungswinkel 64 aufweist. Dieser Neigungswinkel bestimmt, ab welcher Neigung der Austragvorrichtung die Kugel in der Kurvenbahn 62 geführt, auf der Rampe zu rollen und schließlich in die andere, äußere Endlage, die durch die Rastmulde 65 gebildet wird, gelangt. In der Rastmulde 65 liegt die Kugel 61 gehalten zwischen dem Führungsring 60, der fest an dem Betätigungselement 50 befestigt ist und der Anschlagsfläche 16 des Gehäuses 11. Da der Durchmesser der Kugel wenigstens nahezu dem Betätigungsweg eines Betätigungshubes entspricht, wird dann, wenn sich die Kugel 61 in dieser Lage befindet, eine Betätigung des Betätigungselementes gesperrt. Statt drei Kurvenbahnen 62 können auch zwei oder vier Kurvenbahnen vorgesehen sein. Werden zwei Kurvenbahnen vorgesehen, so müssen diese so ausgebilet sein, daß sie doppelseitig arbeiten, d.h. eine Neigung gegenüber der Vorzugsrichtung, in der die Austragvorrichtung ausgerichtet sein soll - meist die Vertikale -, in einer Richtung auf beiden Seiten erfaßt. Wichtig dabei ist nur, daß die Kurvenbahnen 62 die Neigung in einer beliebigen Richtung gegenüber der Vorzugsrichtung erfassen können, also in wenigstens zwei unabhängige Richtungskomponenten auflösen.

Ferner ist in dem Betätigungselement 50 auch die Steuereinheit 70 angeordnet, die eine Zeiterfassung und eine Erfassung der Betätigungen des Betätigungselementes beinhaltet. Die Steuereinheit ist in der Lage, das Sperrglied 74 zwischen einer Freigabestellung 71 und einer Sperrstellung hin- und herzuschalten. Dies geschieht mittels des Elektromagneten 75, der durch die Steuereinheit 70 ansteuerbar ist und der auf das Sperrglied 74 einwirken kann.

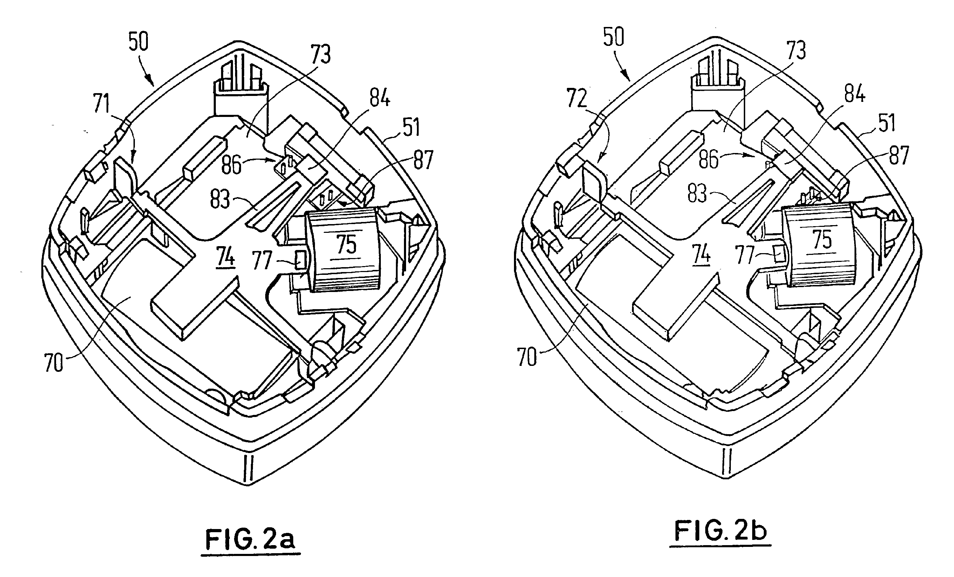

Die Fign. 2a und 2b zeigen eine Ansicht des Betätigungselementes 50 mit der in dem Betätigungselement 50 angeordneten Betätigungssperre. Die Betätigungssperre wird gebildet aus der Steuereinheit 70, dem Elektromagneten 75 sowie dem Sperrglied 74. Vorzugsweise sind diese Elemente auf einer gemeinsamen Grundplatte 73 angeordnet, wobei die Grundplatte im Betätigungselement 50 vorzugsweise durch Verrasten befestigt wird.

Die Fig. 2a zeigt das Sperrglied 74 in der Freigabestellung 71 der Betätigungssperre, während in der Fig. 2b, die ansonsten mit der Fig. 2a übereinstimmt, das Sperrglied 74 in der Sperrstellung 72 der Betätigungssperre.

Die Energieversorgung, die in der Zeichnung nicht dargestellt ist, erfolgt über eine Batterie, vorzugsweise eine Knopfzelle oder Ähnliches, und ist zum Beispiel unterhalb der Grundplatte 73 angeordnet. Mit einem Kondensator kann die Energieversorgung gepuffert werden, was beispielsweise die Aufrechterhaltung der Speicherwerte bei einem Batteriewechsel oder aber eine letztmalige Betätigung des Betätigungselementes 50 nach Ausfall der Versorgung durch die Batterie ermöglicht. Die Grundplatte 73 ist dabei vorzugsweise gleichzeitig als Platine ausgebildet, die die entsprechenden elektrischen Leitungen aufweist. Dazu zählen insbesondere die elektrischen Verbindungen zwischen der Steuereinheit 70 und dem Elektromagneten 75. Der Elektromagnet 75 ist ebenfalls auf der Grundplatte 73 angeordnet. Er wirkt auf einen an dem Sperrglied 74 ausgebildeten Magnetkörper 77 ein. Der Magnetkörper 77 dient dazu, unter dem Einfluß der zwischen Magnetkörper 77 und dem als Stromrelais ausgebildeten Elektromagneten 75 wirkenden elektromagnetischen Kräfte eine Umschaltbewegung des Sperrgliedes 74 zu erzeugen. Das Sperrglied 74 wird dabei in der dargestellten Ausführungsform durch Verschwenken um seine Mittelachse von der Freigabestellung 71 in die Sperrstellung und umgekehrt verbracht.

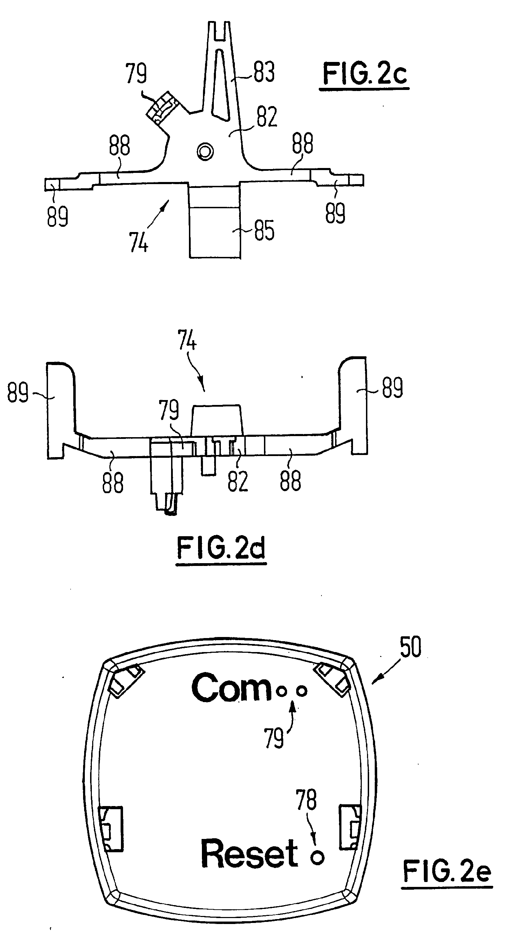

Eine Ausschnittszeichnung des Sperrgliedes 74 ist in Aufsicht und in Seitenansicht in den Fign. 2c bzw. 2d dargestellt.

Zur Erfassung der Lage des Sperrgliedes 74 ist an ihm ein Haltearm 83 ausgebildet, an dessen Ende ein Kontaktstück 84 angeordnet ist. Auf der Grundplatte 73 sind beidseitig des Kontaktstückes 84 erste bzw. zweite Kontaktstiftpaare 86, 87 angeordnet.

In der in Fig. 2a dargestellten Freigabestellung 71 wird mittels des Kontaktstückes 84 die elektrische Verbindung zwischen den beiden Kontaktstiften des zweiten Kontaktstiftpaares 87 geschlossen und somit ein Signal für die Steuereinheit 70 generiert, das anzeigt, daß sich das Sperrglied 74 tatsächlich in der Freigabestellung 71 befindet. Wird das Sperrglied 74 durch die Wirkung des Elektromagneten 75 in die in Fig. 2b dargestellte Sperrstellung verbracht, so wird die Kontaktierung des zweiten Kontaktstiftpaares 87 über das Kontaktstück 84 unterbrochen und am Ende der Betätigung die elektrische Verbindung der Kontaktstifte des ersten Kontaktstiftpaares 86 über das Kontaktstück 84 hergestellt. Somit wird nunmehr ein Signal für die Steuereinheit 70 generiert, das signalisiert, daß sich die Grundplatte 73 in der Sperrstellung 72 befindet. Zugleich ist es möglich, die Sperrkörper 89 an ihrer gehäuseseitigen Fläche mit einer Farbmarkierung zu versehen, die über ein gehäuseseitiges Sichtfenster erfaßbar ist, und eine optische Information über die Schaltstellung des Sperrgliedes liefert (z.B. grün = Freigabe-, rot = Sperrstellung).

Die Fig. 2e zeigt die Ansicht des Betätigungselementes 50 von unten, der Gehäuseaußenseite des Betätigungselementes 50. Diese weist an ihrer Bodenfläche 58 zum einen den Schalter 78 und zum anderen die Schnittstelle 79 auf. Über den Schalter 78, der insbesondere als versenkt angeordneter Tastschalter ausgebildet sein kann, dessen Betätigung beispielsweise nur mittels eines Hilfsmittels, vorzugsweise einem spitzen Gegenstand, beispielsweise einer Bleistiftspitze, durchgeführt werden kann, angeordnet. Des weiteren ist auf der Bodenfläche 58 auch die Schnittstelle 79 angeordnet. Die Schnittstelle 79 dient der Kontaktierung eines Informationsmittels mit der Steuereinheit 70. Bei dem Informationsmittel kann es sich entweder um ein passives Bauteil, im einfachsten Fall um eine Kontaktierungsbrücke oder aber auch um eine Eingabe-/Ausgabeeinheit, wie zum Beispiel einen PC, handeln, über den Daten, vorzugsweise Parameter für die Funktion der Steuereinheit 70, an diese übertragen werden können und auch Informationen aus der Steuereinheit ausgelesen werden können. Bei den Parametern, die an die Steuereinheit 70 übertragen werden können, handelt es sich insbesondere um den Wert des ersten Zeitintervalls, der beginnt, wenn eine Betätigung des Betätigungselementes erfolgt und das den Zeitraum festlegt, der vor der nächsten Betätigung des Betätigungselementes verstreichen muß, damit kein Sperren der Betätigung erfolgt. Ferner kann auch das zweite Zeitintervall als Parameter in die Steuereinheit 70 übertragen werden. Das zweite Zeitintervall bestimmt die Zeit, während der nach Betätigung des Schalters 78 eine vorgegebene Anzahl von Betätigungen des Betätigungselementes durchgeführt werden kann, ohne daß die Betätigungssperre eingreift. Ebenso ist diese Anzahl von Betätigungen, die ohne Eingreifen der Betätigungssperre nach Betätigen des Schalters 78 durchführbar ist, über die Schnittstelle 79 vorgebbar. Bei der Schnittstelle 79 kann es sich also insbesondere um eine Steckverbindung eines Datenbusses für die Steuereinheit 70 handeln. Als Datenbus kommen bevorzugt Zweidraht-Datenbusse in Betracht. Des weiteren ist es möglich, über die Schnittstelle 79 auch Informationen aus der Steuereinheit 70 auszulesen. Es können beispielsweise die Anzahl der erfolgten Betätigungshübe sowie die Anzahl der Betätigungen des Schalters 78 erfaßt werden. Ferner wäre es auch möglich, zu erfassen, wie oft das Betätigungselement vor Ablauf des ersten Zeitintervalls nach der vorhergehenden Betätigung des Betätigungselementes erfolgt ist. Diese Information kann insbesondere als Maß für das Bedürfnis des Patienten gewertet werden, eine höhere Wirkstoffdosis verabreicht zu bekommen. Welche Parameter vorgebbar sind und welche Informationen aus der Steuereinheit 70 ausgelesen werden, kann entsprechend den Bedürfnissen festgelegt werden. Dies ist lediglich bei der Gestaltung der Steuereinheit 70 entsprechend zu berücksichtigen.

In einfacherer Ausbildung kann das Informationselement auch lediglich eine Kontaktbrücke zur elektrischen Kontaktierung zwischen den beiden Einzellitzen, die an der Schnittstelle 79 enden, ausgebildet sein. Vorzugsweise liegt die Kontaktbrücke in Form eines gesondert geformten Steckers vor, der zum Beispiel nur an einen eingeschränkten Personenkreis, wie Krankenschwestern und Apotheker, ausgegeben wird. Wird die Präsenz des Brückensteckers an der Schnittstelle 79 verlangt, wenn der Schalter 78 betätigt wird, so kann in dieser Weise sichergestellt werden, daß nicht Unbefugte eine Anzahl von Betätigungen des Betätigungselementes 50 durchführen können, ohne daß die Betätigungssperre eingreift. Dies ist eine Maßnahme im Sinne der Erhöhung der Bediensicherheit. Dies kann es aber erforderlich machen, daß die erste Inbetriebnahme der Ausgabevorrichtung ebenfalls durch eine dazu befugte Person erfolgen muß.

Die Fign. 2c und 2d zeigen die Aufsicht und die Seitenansicht des Sperrgliedes 74. Das Sperrglied 74 weist in seinem Zentrum 82 die Form einer Scheibe auf, die um die Mittelachse 80 drehbar gelagert ist. Von dem Zentrum ragen einerseits der Haltearm 83, der an seinem Ende zur Aufnahme des Kontaktstückes 84 ausgebildet ist und auch der Permanent-Magnetkörper 77 nach außen hin ab. Gegenüber von Magnetkörper 77 und Haltearm 83 ist die Ausgleichsmasse 85 ausgebildet, die für eine wenigstens annähernde Wuchtung des Sperrgliedes 74 bzgl. der Mittelachse 80 und somit eine leichtgängige Betätigbarkeit des Sperrgliedes 74 sorgt. Ferner ragen zwei auf einer exzentrisch zur Mittelachse 80 verlaufenden Achse angeordnete Arme 88 von dem Zentrum 82 ab. Am Ende der Arme 88 sind die Sperrkörper 89 ausgebildet. Die Sperrkörper ragen dabei parallel zur Mittelachse 80 im Betätigungselement 50 nach oben, heraus aus der Ebene des Sperrgliedes 74. Dabei entspricht die Höhe der Sperrkörper 89 dem Weg eines Betätigungshubes des Betätigungselementes 50. In der Freigabestellung 71 des Sperrgliedes 74 ist es möglich, beispielsweise in einer Führungsnut, die Sperrkörper 89 in das Gehäuse 11 hineinzuführen. In der Sperrstellung 72 befinden sich die Sperrkörper 89 in einer Lage, in der sie den Zwischenraum zwischen dem unteren Ende des Gehäuses 11 mit seiner Anschlagfläche 16 und einer entsprechend ausgebildeten Fläche am Betätigungselement ausfüllen. Dadurch wird ein Verschieben des Betätigungselementes um den Weg des Betätigungshubes der Austragvorrichtung gesperrt. Dadurch ist ein Betätigen der Austragvorrichtung unmöglich. Dabei ist zu beachten, daß eine Austragvorrichtung, wie sie hier Anwendung findet, insbesondere also ein Pump-Zerstäuber, einen gewissen Leerweg hat. Um maximal diesen Leerweg können die Sperrkörper 98 kürzer sein als der Weg des Betätigungshubes des Betätigungselementes 50. Das Sperrglied 74 wird durch Verschwenken um die Mittelachse 80 in die Sperrstellung 72 und wieder zurück in die Freigabestellung 71 verfahren. Diese Bewegung ist, zumindest soweit die Sperrkörper nicht im Gehäuse kraftschlüssig gehalten werden, beispielsweise wenn eine Person das Betätigungselement 50 betätigt und der Sperrkörper 89 sich in seiner Sperrstellung 72 befindet, fast kraftfrei geschehen. Daher ist ein Elektromagnet, der geringe Kräfte erzeugt, ausreichend, um sehr rasch die Schaltstellung des Sperrgliedes 74 zu ändern. Es ist dabei kein großer Kraftaufwand und auch kein großer Energieaufwand erforderlich. Insbesondere kann ein Umschalten in dem kurzen Zeitraum stattfinden, in dem der Leerweg des Betätigungselementes 50 bei einer Betätigung zurückgelegt wird. Dann ist es auch bei einem in Ruhelage monostabil in der Freigabestellung 71 befindlichen Sperrglied 74 energiesparend möglich, durch kurze Betätigung des Elektromagneten die Sperrstellung zu erzeugen.

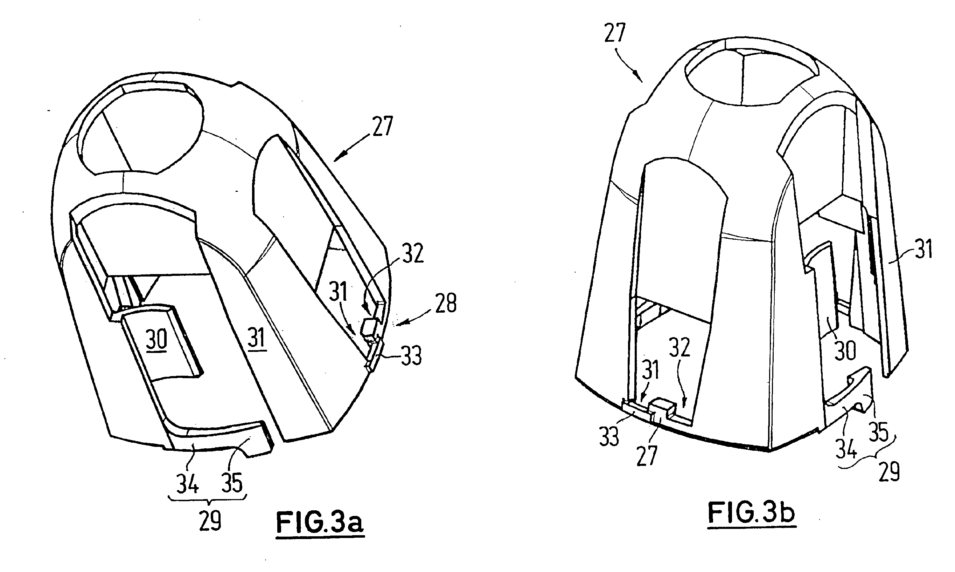

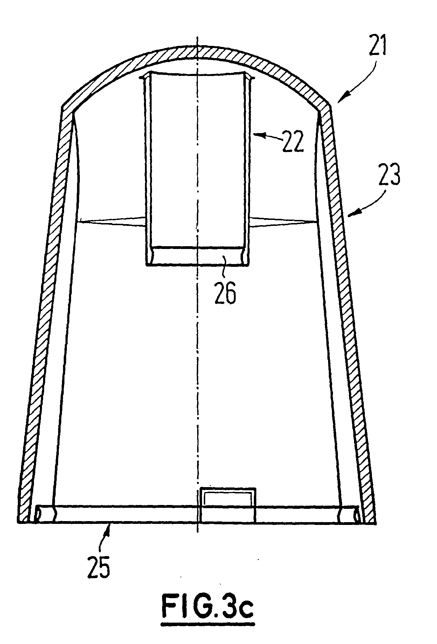

Die Fign. 3a und 3b zeigen aus zwei unterschiedlichen Perspektiven eine Ansicht des Innenteils 27 der Verschlußkappe 20. Die Verschlußkappe 20 wird gebildet aus dem Innenteil 27 und dem in Fig. 3c dargestellten Außenteil 21. Das Innenteil 27 wird gebildet aus einem Grundkörper 31. Der Grundkörper 31 trägt dabei alle für die Funktion der Verschlußkappe ausgebildeten Elemente. Zur Ausbildung der Kulissenführung 28 für den am Gehäuse befindlichen Gleitstein 19 ist ein Freiraum 31 vorgesehen. Der Freiraum 31 führt vorbei an einem Steg. Nachdem der Steg überfahren wurde, ist es möglich, die Verschlußkappe um einen Winkel - der vorzugsweise kleiner als 90° ist -, im dargestellten Beispiel beträgt der Winkel ca. 20°, zu verdrehen. Dann kann der Gleitstein in die Mulde 32 "fallen". In der Mulde 32 ist der Gleitstein 19 so gehalten, daß ein Verdrehen der Verschlußkappe selbst direkt aus dieser Lage nicht möglich ist. Damit der Gleitstein 19 sicher in die Mulde 32 gelangt, ist an dem Innenteil 27 der Kraftspeicher 29 in Form eines verbiegbaren Materialelementes ausgebildet. Dieses Materialelement ist im Bereich des Schaftes 34 elastisch verformbar. Der Kopf 35 ragt dabei in den Innenraum des Innenteils. Er gelangt mit dem Gehäuse 11 in Anlage. Dadurch, daß die Innenseite des Kopfes ebenso angeschrägt ist, wie das Gehäuse 11 in diesem Bereich angeschrägt ist, wird der Kopf nach außen gedrückt, wodurch eine Kraft aufgebaut wird, wenn der Innenteil bzw. die Verschlußkappe auf das Gehäuse 11 aufgesetzt wird. Die Schrägung bewirkt, daß dann, wenn keine Kraft mehr von außen auf die Verschlußkappe 20 einwirkt, die elastische Verformung Tendenz hat, sich zurückzubilden und diese Kraft, die am Gehäuse abgestützt ist, die Verschlußkappe in Löserichtung auf dem Gehäuse 11 nach oben schiebt. Somit gelangt aufgrund des zwischenzeitlich erfolgten Verdrehens der Verschlußkappe 20 der Gleitstein 19 des Gehäuses in die Mulde 32. Zusätzlich ist am Innenteil 27 noch das Wirkelement 30 ausgebildet. Das Wirkelement 30 ist ebenfalls ein Materialsteg, der in gewissen Grenzen elastisch verformbar ist. Das Wirkelement 30 gerät beim Verdrehen der Verschlußkappe auf dem Gehäuse in Anlage mit dem unrund geformten Gehäuse 11. Während des Verdrehens wird dabei das Wirkelement 30 nach außen gedrückt und leicht elastisch verformt. Somit wird eine dem Öffnen der Verschlußkappe entgegenwirkende Kraft aufgebaut. Gleichzeitig muß auch eine bestimtme Kraft aufgewandt werden, um die Verschlußkappe auf das Gehäuse aufzusetzen. Dies dient dadurch der Bediensicherheit, daß der Benutzer der Austragvorrichtung gezwungen ist, bei der Betätigung zum Öffnen eine Mindestkraft aufzubringen und auch bei dem Verschließen des Gehäuses mit der Verschlußkappe eine Kraft aufgebracht wird.

Vorteilhafterweise sind auf zwei gegenüberliegenden Seiten Kulissenführungen 28 und dazwischen jeweils Kraftspeicher 29 und/oder Wirkelement 30 ausgebildet.

Dabei ist es aus fertigungstechnischen Gründen einfach, die Verschlußkappe aus dem Innenteil 27 und aus dem Außenteil 21 herzustellen. Es ist auch möglich, nur eine einteilige Verschlußkappe vorzusehen, die dann aber komplizierter geformt ist oder nach außen hin nicht durchgängig eine geschlossene glatte Oberfläche aufweist.

Die Fig. 3c zeigt das Außenteil 21. Das Außenteil 21 weist eine geschlossene glatte Außenfläche 23 auf. In das Innere des Außenteils 21 wird das Innenteil 27 eingesetzt und dort form- oder kraftschlüssig befestigt. Ferner weist das Innere des Außenteils 21 noch den als Hohlzylinder ausgestalteten Formabschnitt 22 auf. Der Formabschnitt 22 stößt mit einem Ende an die Innenseite der geschlossenen Fläche 23. An seinem anderen Ende ist die dichtende Anlage 26 ausgebildet, die sich an das Gehäuse 11 so anlegt, daß die Austragöffnung 12 umschlossen wird.

Darüber hinaus ist an dem Außenteil 21 noch der untere Rand 25 ausgebildet. An dem unteren Rand 25 kann sich, bei auf das Gehäuse 11 aufgesetzter Verschlußkappe, ein in der Zeichnung nicht dargestellter Dorn abstützen. Der Dorn ist dabei durch eine im Gehäuse vorgesehene Öffnung 11 herausgeführt und fest mit dem Betätigungselement 50 verbunden. Diese Maßnahme verhindert, daß eine Betätigung des Betätigungselementes 50 bei verschlossener Verschlußkappe erfolgt. Dies erhöht die Sicherheit und verhindert insbesondere im Zusammenwirken mit der Zeitsperre während des ersten Zeitintervalls nach einer Betätigung des Betätigungselementes 50 die Betriebssicherheit. Eine Betätigung des Betätigungselementes 50 ist erst dann möglich, wenn die Verschlußkappe abgenommen wurde.

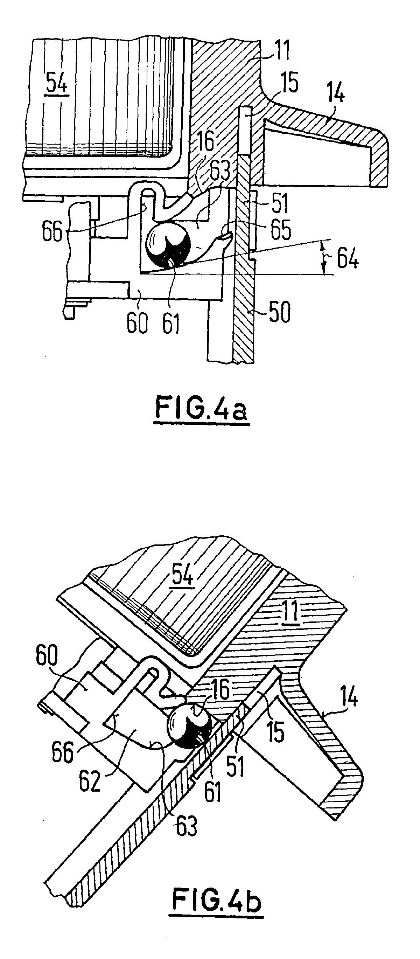

Die Fign. 4a und 4b zeigen in zwei unterschiedlichen Stellungen die Funktion des Führungsringes 60. Der Führungsring 60 ist am Betätigungselement 50 befestigt und im Bereich dessen oberen Endes, also dem gehäuseseitigen Ende, angeordnet. Das Gehäuse 11 weist die Ringnut 15 auf, in die der Tauchsteg 51 des Betätigungselementes 50 während einer Betätigung eintaucht. Dabei ist der Führungsring 60 so angeordnet, daß die Kurvenbahn 62 für die in er der Kurvenbahn 62 geführte Kugel 61 an der Außenseite des Betätigungselementes 50 mündet und zwar unmittelbar unterhalb der Anschlagfläche 16 am Gehäuse 11. Dabei kann die Anschlagfläche 16 so geformt sein, daß sie einen Teil der Führung der Kugel 61 in der Führungsbahn übernimmt.

Die Fig. 4a zeigt die Situation, in der sich die Austragvorrichtung in einer Lage befindet, in der ein Austragen möglich sein soll. Vorzugsweise ist dies dann erwünscht, wenn sich die Austragvorrichtung nicht mehr als um einen vorgegebenen Winkel, der vorzugsweise in einem Bereich zwischen 30 und 35° liegt, bzgl. der Vertikalen (als Vorzugsrichtung) geneigt ist. Die Kugel 61 ist in der Kurvenbahn 62 des Führungsringes 60 geführt. Die Kurvenbahn 62 weist eine schiefe Ebene 63 auf, so daß in nahezu vertikaler Lage die Kugel 61 sich am innenseitigen Endanschlag 66 befindet. In dieser Stellung ist es möglich, einen Betätigungshub des Betätigungselementes durchzuführen. Dazu muß nur das Betätigungselement um den Betätigungsweg relativ zum Gehäuse 11 bewegt werden.

Sobald die Neigung der Austragvorrichtung einen durch die Neigung 64 der schiefen Ebene 63 vorgegebenen Winkel bzgl. der Vertikalen übersteigt, rollt die Kugel in der Kurvenbahn 62. Sie rollt dabei so weit, bis sie am äußeren Ende der Kurvenbahn angekommen ist und am Betätigungselement 50 anschlägt. Sie wird dann in der Rastmulde 65, die an der Kurvenbahn 62 ausgebildet ist, gehalten. Sie befindet sich nun zwischen der Rastmulde 65, also dem Führungsring 60 und der seitens des Gehäuses 11 ausgebildeten Anschlagfläche 16. Da die Kugel 61 einen Durchmesser hat, der annähernd dem Weg eines Betätigungshubes entspricht, blockiert sie die Durchführung eines Betätigungshubes, da der erforderliche Freiraum zwischen Rastmulde 65 und Anschlagfläche 16 durch die Kugel ausgefüllt wird. Somit wird eine Betätigung des Betätigungselementes 50 verhindert, solange sich die Neigung der Austragvorrichtung gegenüber der Vorzugsrichtung nicht innerhalb einer vorgegebenen Winkellage befindet. Die Sperrstellung der Kugel 61 ist in der Fig. 4b dargestellt.

Dies stellt sicher, daß bei jedem Austraghub aus dem Behälter 54 die gleiche Menge an Medium ausgetragen wird. Dies ist insbesondere dann besonders wichtig, wenn eine Zeitsperre vorgesehen ist, die eine nachfolgende Betätigung des Betätigungselementes 50 sperrt. Wird nun bei einer Betätigung nur eine unzureichende Teilmenge des Mediums ausgetragen, so kann nicht durch eine zweite Betätigung oder eine zweite Teilbetätigung die Restmenge oder auch eine vollständige Austragmenge noch ausgetragen werden. Dieses Problem stellt sich insbesondere dann, wenn zum Austragen eine Saugpumpe verwendet wird. Bei derartigen Saugpumpen ist es möglich, daß dann, wenn die Neigung des Behälters einen bestimmten Winkel überschreitet, keine vollständige Ansaugung von Medium stattfindet, sondern ein Teilvolumen Nebenluft angesaugt und ausgetragen wird.

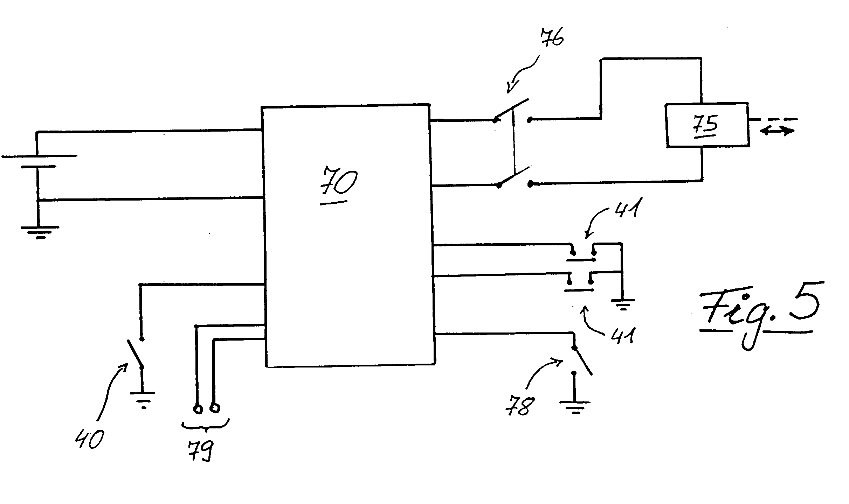

Die Fig. 5 zeigt den Schaltplan für eine erfindungsgemäße Austragvorrichtung für Medien mit einer Betätigungssperre, die durch eine Steuereinheit ansteuerbar ist. Zur Betätigung des Betätigungselementes ist der Elektromagnet 75 angeordnet und von der Steuereinheit 70 ansteuerbar.

Wenn die Steuereinheit zum ersten Mal mit Strom versorgt wird, also eine Batterie zur Stromversorgung seitens des Betätigungselementes anmontiert wird, oder vom Benutzer ein Kontaktschutz von der Batterie oder den Kontaktelementen, die den elektrischen Kontakt mit der Batterie herstellen, entfernt wird, erlaubt die Steuereinheit die Durchführung einer vorgegebenen Anzahl von Betätigungen des Betätigungselementes 50, ohne daß eine Betätigungssperre wirksam wird. Diese Betätigungen dienen der Initialisierung der Austragvorrichtung in der schon beschriebenen Weise.

Dabei wird die Betätigung eines Betätigungselementes über den Dosiersensor 40 erfaßt. Dieser Dosiersensor ist so im Betätigungselement 50 angeordnet, daß eine Betätigung des Betätigungselementes 50 erfaßt wird, die ein bestimmtes Maß überschreitet. Der Sensor ist vorzugsweise als Schalter bzw. Tastschalter ausgebildet, der durch Relativlageveränderung zwischen Betätigungselement 50 und Gehäuse 11 geschaltet wird.

Wird das Betätigungselement betätigt, so wird der Dosiersensor 40 betätigt, ein entsprechendes Schaltsignal wird in der Steuereinheit 70 generiert. Daraufhin wird überprüft, ob seit der vorhergehenden Betätigung des Betätigungselementes 50 zumindest ein vorgegebenes Zeitintervall überschritten wurde. Ist dies nicht der Fall, so wird nun der Elektromagnet dahingehend aktiviert, daß die Betätigungssperre mit ihrem Sperrglied 74 in die Sperrstellung 72 verbracht wird. Andernfalls wird das Relais so angesteuert, daß das Sperrglied 74 in die Freigabestellung 71 verbracht wird. Wird eine Betätigung des Betätigungselementes 50 ermöglicht, so wird gleichzeitig der Zeitzähler für die Überwachung des Zeitintervalls seit der letzten Betätigung der Austragvorrichtung wieder auf Null zurückgesetzt. Selbstverständlich kann die Überwachung auch einen Zeitzähler umfassen, der nicht inkriminiert, sondern auf Null zurückdekriminiert. Gleichzeitig mit dem Verbringen des Sperrgliedes 74 in die Freigabestellung 71 bzw. die Sperrstellung 72 kann ein Alarmsignal über einen Signalgeber erzeugt werden. Vorzugsweise ist das Signal ein akustisches Signal.

Diese Vorgehensweise entspricht der Ausbildung des Sperrgliedes 74 als bistabilem Element. Ist dieses aber als monostabiles Element ausgebildet und zum Beispiel durch einen Kraftspeicher entweder in der Sperrstellung 72 oder der Freigabestellung 71 gehalten, so muß eine Betätigung des Elektromagneten nur dann erfolgen, wenn ein Umschalten des Sperrgliedes erfolgen soll.

Eine Vorspannung des monostabilen Sperrgliedes 74 in der Freigabestellung 71 hat den Vorteil, daß bei Ausfall der Elektronik ein Austrag durch Betätigung des Betätigungselementes 50 erfolgen kann, dann jedoch ein unkontrollierter, den Abstand des ersten Zeitintervalls nicht beachtender Austrag möglich ist.

Im Gegensatz dazu verhindert eine Vorspannung des monostabilen Sperrgliedes 74 in der Sperrstellung 72 diesen unkontrollierten Austrag im Fehlerfall, allerdings ist dann auch ein das erste Zeitintervall beachtender Austrag wegen der Sperrstellung 72 des Sperrgliedes 74 unmöglich. Insbesondere in diesem Fall ist es vorteilhaft, mechanische Mittel (entfernbares Sicherungselement) vorzusehen, die ggf. unter Inkaufnehmen einer irreversiblen Zerstörung, ein wenigstens einmaliges Betätigen des Betätigungselementes 50 ermöglichen.

Die Freigabestellung 71 bzw. die Sperrstellung 72 des Sperrgliedes 74 wird über die Kontrollsensoren 41 erfaßt. Die Kontrollsensoren 41 werden aus den ersten bzw. zweiten Kontaktstiftpaaren gebildet, die vom Kontaktstück 84 kontaktiert werden und so die Lage erfassen. Über den Schalter 78 kann ein neuer Start generiert werden, bei dem die vorgegebene Anzahl von Betätigungen des Betätigungselementes 50 ohne Eingreifen der Betätigungssperre durchgeführt werden kann. Über die Schnittstelle 79 kann zusätzlich auf die Steuereinheit zugegriffen und eine Datenkommunikation durchgeführt werden. Insbesondere können die Parameter der Steuereinheit 70 eingestellt werden und erfaßte Daten über die Benutzung der Austragvorrichtung ausgelesen werden.

## Patentansprüche

1. Austragvorrichtung für Medien, insbesondere für das Versprühen eines vorzugsweise einen pharmazeutischen Wirkstoff enthaltenden Fluids, mit einem Gehäuse (11), das eine Austragöffnung (12) zum Austragen des Mediums aufweist, mit einer abnehmbaren Verschlußkappe (20), die auf dem Gehäuse (11) durch Verrasten befestigbar ist und in der verrasteten Lage die Austragöffnung (12) überdeckt, dadurch gekennzeichnet, daß die Verrastung so ausgebildet ist, daß ein Abnehmen der Verschlußkappe (20) nur durch eine Betätigung der Verschlußkappe (20) möglich ist, die eine Abfolge von wenigstens zwei Teilbetätigungen in von der vorherigen Teilbetätigung unterschiedlicher, voneinander unabhängiger Betätigungsrichtung, umfaßt.

2. Austragvorrichtungnach Anspruch 1, dadurch gekennzeichnet, daß die erste Teilbetätigung in einer dem Entfernen der Verschlußkappe (20) vom Gehäuse 11) entgegengesetzte Betätigungsrichtung erfolgt.

3. Austragvorrichtung nach Anspruch 1 oder 2, dadurch gekennzeichnet, daß die zweite Teilbetätigung eine Rotation um die Längsachse der Verschlußkappe (20) ist.

4. Austragvorrichtung nach Anspruch 3, dadurch gekennzeichnet, daß der Drehwinkel der Rotation der zweiten Teilbetätigung begrenzt ist, vorzugsweise auf einen Winkel kleiner 90°, insbesondere auf einen Winkel zwischen 10 und 45°.

5. Austragvorrichtung nach einem der Ansprüche 1 bis 4, dadurch gekennzeichnet, daß die Verrastung als eine zwischen Verschlußkappe (20) und Gehäuse (11) ausgebildeten Kulissenführung ausgebildet ist.

6. Austragvorrichtung nach Anspruch 5, dadurch gekennzeichnet, daß die Kulisse der Kulissenführung (28) in der Verschlußkappe (20) ausgebildet ist.

7. Austragvorrichtung nach Anspruch 5 oder 6, dadurch gekennzeichnet, daß der Verlauf der Kulisse (28) dem Kulissenverlauf eines Bajonettverschlusses entspricht.

8. Austragvorrichtung nach einem der Ansprüche 5 bis 7, dadurch gekennzeichnet, daß mehrere, regelmäßig beabstandete Kulissenführungen (28) zwischen Verschlußkappe (20) und Gehäuse (11) ausgebildet sind.

9. Austragvorrichtung nach einem der Ansprüche 1 bis 8, dadurch gekennzeichnet, daß zwischen Verschlußkappe (20) und Gehäuse (11) wirkende Kraftspeicher (29) vorgesehen sind, wobei die Kraftspeicher (29) im Sinne eines Aufrechterhaltens der Verrastung wirken.

10. Austragvorrichtung nach Anspruch 9, dadurch gekennzeichnet, daß die Kraftspeicher (29) aus an der Verschlußkappe (20) ausgebildeten, elastisch verformbaren Stegen (33) gebildet sind, die mit einer entsprechend geformten Fläche des Gehäuses (11) zusammenwirken.

11. Austragvorrichtung nach einem der Ansprüche 1 bis 10, dadurch gekennzeichnet, daß an der Verschlußkappe (20) Wirkelemente (30) ausgebildet sind, die wenigstens der zweiten Teilbetätigung der Verschlußkappe (20) überwindbare entgegenwirkende Kräfte erzeugen.

12. Austragvorrichtung nach einem der Ansprüche 8 bis 11, dadurch gekennzeichnet, daß wenigstens ein Kraftspeicher (29) und/oder ein Wirkelement (30) zwischen zwei Kulissenführungen (28) angeordnet sind.

13. Austragvorrichtung nach einem der Ansprüche 1 bis 12, dadurch gekennzeichnet, daß die Verschlußkappe (20) zweiteilig ausgebildet ist, wobei ein Innenteil (27) in ein Außenteil (21) eingesetzt ist und wobei das Außenteil (21), abgesehen von der dem Gehäuse (11), zugewandten Öffnung eine geschlossene Oberfläche aufweist.

14. Austragvorrichtung nach Anspruch 13, dadurch gekennzeichnet, daß Kulissenführung (28) und/oder Kraftspeicher (29) und/oder Wirkelemente (30) an dem Innenteil (27) ausgebildet sind.

15. Austragvorrichtung nach Anspruch 13 oder 14, dadurch gekennzeichnet, daß an der Verschlußkappe (20) insbesondere am Außenteil (21) ein Formabschnitt (22) ausgebildet ist, wobei der Formabschnitt (22) die Austragöffnung (12) umschließt und dichtend am Gehäuse (11) anliegt.
